# EUROPEAN PATENT SPECIFICATION

(11) **EP 2 644 695 B1**
(45) Date of publication and mention of the grant of the patent: **01.08.2018**
(21) Application number: 13160628.7
(22) Date of filing: 22.03.2013
(51) Int. Cl.: C12N 5/077, C12N 5/074, C12N 5/0775, A61F 2/30, A61L 27/38

(54) **CULTURED CARTILAGE TISSUE MATERIAL**
KULTIVIERTES KNORPELGEWEBEMATERIAL
MATÉRIAU DE TISSU CARTILAGINEUX EN CULTURE

(30) Priority: 30.03.2012 JP 2012079623
(43) Date of publication of application: 02.10.2013
(73) Proprietor: GC Corporation, Bunkyo-ku Tokyo 113-0033 (JP)
(72) Inventor: YAMANAKA, Katsuyuki, Tokyo, 174-8585 (JP); SAKAI, Yuuhiro, Tokyo, 174-8585 (JP); YAMAMOTO, Katsushi, Tokyo, 174-8585 (JP); SHIGEMITSU, Yusuke, Tokyo, 174-8585 (JP)
(74) Representative: Beck Greener

(56) References cited:
- EP-A1- 2 080 800
- US-B1- 6 454 811
- C. R. LEE ET AL: "Effects of a cultured autologous chondrocyte-seeded type II collagen scaffold on the healing of a chondral defect in a canine model", JOURNAL OF ORTHOPAEDIC RESEARCH, vol. 21, no. 2, 1 March 2003 (2003-03-01), pages 272-281, XP055062642, ISSN: 0736-0266, DOI: 10.1016/S0736-0266(02)00153-5
- RONNY MAIK SCHULZ ET AL: "Cartilage tissue engineering and bioreactor systems for the cultivation and stimulation of chondrocytes", EUROPEAN BIOPHYSICS JOURNAL ; WITH BIOPHYSICS LETTERS, SPRINGER, BERLIN, DE, vol. 36, no. 4-5, 23 February 2007 (2007-02-23), pages 539-568, XP019513508, ISSN: 1432-1017, DOI: 10.1007/S00249-007-0139-1
- ROBERT L. MAUCK ET AL: "Influence of Seeding Density and Dynamic Deformational Loading on the Developing Structure/Function Relationships of Chondrocyte-Seeded Agarose Hydrogels", ANNALS OF BIOMEDICAL ENGINEERING, vol. 30, no. 8, 1 September 2002 (2002-09-01), pages 1046-1056, XP055062633, ISSN: 0090-6964, DOI: 10.1114/1.1512676
- ANNA ASANBAEVA ET AL: "Mechanisms of cartilage growth: Modulation of balance between proteoglycan and collagen in vitro using chondroitinase ABC", ARTHRITIS & RHEUMATISM, vol. 56, no. 1, 1 January 2007 (2007-01-01), pages 188-198, XP055019359, ISSN: 0004-3591, DOI: 10.1002/art.22298
- LEE JIN WOO ET AL: "Chondrogenic differentiation of mesenchymal stem cells and its clinical applications", YONSEI MEDICAL JOURNAL, YONSEI UNIVERSITY, KI, vol. 45, no. Suppl. S, 30 June 2004 (2004-06-30), pages 41-47, XP002511546, ISSN: 0513-5796
- WANG W ET AL: "The restoration of full-thickness cartilage defects with BMSCs and TGF-beta 1 loaded PLGA/fibrin gel constructs", BIOMATERIALS, ELSEVIER SCIENCE PUBLISHERS BV., BARKING, GB, vol. 31, no. 34, 1 December 2010 (2010-12-01), pages 8964-8973, XP027381059, ISSN: 0142-9612 [retrieved on 2010-09-06]
- WU ET AL: "Proliferation of chondrocytes on porous poly(dl-lactide)/chitosan scaffolds", ACTA BIOMATERIALIA, ELSEVIER, AMSTERDAM, NL, vol. 4, no. 1, 22 November 2007 (2007-11-22), pages 76-87, XP022357446, ISSN: 1742-7061

## Description

### Technical Field

The present invention relates to a cultured cartilage tissue material to be used for repairing a damaged site of cartilage or bone caused by a cartilage-related disease, an accident, or the like.

### Background Art

So-called regenerative medicine has been employed to treat a damaged site such as a defect of cartilage caused by a cartilage-related disease such as osteoarthritis, by an athletic injury, by an accident, or the like. The regenerative medicine is a medical technology to restore biological tissue that can no longer be recovered by the healing capability of the living body, by using a cell, a carrier, a growth factor and so on, so that the form and function of the tissue are restored to the nearly same state of an original tissue.

In order to regenerate cartilage tissue, a cartilage tissue regeneration material in which chondrocytes or stem cells to differentiate into chondrocytes have been seeded and cultured, is necessary.

Various forms of the method to implant an artificial cartilage tissue prepared by culturing have been studied. A typical example thereof is given in Non-Patent Document 1, which discloses a method in which chondrocytes harvested from a patient are cultured in monolayer to amplify them, and thereafter the cells are made into a cell suspension to be implanted to a defect site of cartilage. This method includes covering the implanted site with a periosteum patch after the implantation so that the implant material does not dissipate.

In addition, Patent Document 1 discloses a method in which chondrocytes harvested from a patient are seeded in a scaffold material made of atelocollagen, are cultured therein, and thereafter the paste-form collagen gel made therefrom is implanted.

Patent Document 2 discloses a production method of an artificial cartilage tissue, the method comprising: isolating cartilage-derived cells from cartilage taken from a mammal; and culturing thus isolated cartilage-derived cells under the presence of serum or blood plasma originated from the mammal species.

### Citation List

### Non-Patent Documents

Non-Patent Document 1: Mats Brittberg, "Treatment of Deep Cartilage Defects in the Knee with Autologous Chondrocyte Transplantation", New England Journal of Medicine, USA, Massachusetts Medical Society, 1994, 331, pp. 889-895

### Patent Documents

Patent Document 1: Japanese Patent Application Laid-Open (JP-A) No. 2001-224678
Patent Document 2: JP-A No. 2007-301387

EP 2080800 discloses a porous cell scaffold being capable of seeding cells in a whole small hole structure of a scaffold without applying high force to the cells and almost accurately grasping an amount of cells in the scaffold, and a production method thereof.

### Summary of the Invention

### Problems to be Solved by the Invention

Although the method described in Non-Patent Document 1 has a better effect compared to autonomous healing, the cells are poorly retained, and thus a high healing effect is difficult to attain. Further, this method is to make cartilage by culturing chondrocytes in monolayer; therefore the cartilage thus made is different from that in the living body. In specific, the original characteristics of cartilage partially disappears (the dedifferentiation occurs), thus causing a problem that the implant material is required to go through many things, which are "redifferentiaton" "biosynthesis of a matrix" and "restoration of a defect site", and inevitably requiring time for restoration. As a result, dissipation of the implant cells occurs frequently during treatment period. Furthermore, this method does not enable formation of a cartilage tissue material that is large in volume; therefore it cannot be employed when the cartilage is damaged to a large extent.

The invention described in Patent Document 1 is a combination of: a collagen gel mainly composed of type I collagen; and chondrocytes. As such, a ratio at which type I collagen is mixed in type II collagen being a matrix, is at least 10% or more. Therefore, it is completely different from biological cartilage tissue containing almost no type I collagen. Further, an implanted site needs to be covered with a periosteum patch after the implantation. Furthermore, in this invention, cartilage is used in paste form, which is different from the form of the cartilage in the living body, thus being much weaker than the actual cartilage, taking long time to turn into the actual cartilage, or being difficult to be applied to the cartilage in a loaded part of the joint.

The artificial cartilage tissue described in Patent Document 2 has a drawback that it is different from the actual cartilage as in the cases of other prior techniques. Further, this artificial cartilage tissue is obtained by making a tissue body only of cells and thus does not use a scaffold material. Therefore, when it has a thickness of 2 mm or more, the cells in the central part come to necrose. Accordingly, there is a drawback that a cultured tissue in practical thickness cannot be made. That is, this artificial cartilage tissue has a limitation in the size of the tissue body that can be made; and therefore it is difficult to make a cartilage tissue having a size of 2 mm or more in the thinnest part as required in actual cases.

Accordingly, an object of the present invention is to provide a cultured cartilage tissue material which enables formation of cartilage tissue being substantially the same as the cartilage tissue actually in the living body, and which is large in volume.

### Means for Solving the Problems

The present invention will be described below.

A first aspect of the present invention is a cultured cartilage tissue material to be used for cartilage regeneration comprising chondrocytes, wherein the cultured cartilage tissue material is a mixed body of the chondrocytes in a concentration of 1 × 10⁷ to 1 × 10⁹ cells/cm³, and a bioabsorbable polymer; a thickness of the thinnest part of the cultured cartilage tissue material is 2.2 to 100 mm; a volume ratio between the chondrocytes and the bioabsorbable polymer is 7:3 to 9.5:0.5; a production amount of glycosaminoglycan is 0.001 to 0.2 ng per unit cell; a content ratio between type I collagen and type II collagen is 10:90 to 1:99; and a content of type II collagen is 0.01 to 0.65 mg per 1 mg of dry weight of tissue.

Preferably, an area of the base of the cultured cartilage tissue material is 0.5 cm² to 200 cm².

### Effects of the Invention

According to the present invention, it is possible to make cartilage tissue being substantially the same as the cartilage tissue existing in the living body, therefore enabling shortened treatment period, and also possible to make a large-sized cartilage tissue material. As such, the present invention can be applied in treating a cartilage defect, which has been considered difficult to perform.

### Brief Description of the Drawings

Fig. 1 is a view illustrating a part of a production method S10 of a cultured cartilage tissue material: Fig. 1A shows a placement step; and Fig. 1B shows a cell suspension introduction step.
Fig. 2 is another view illustrating a part of the production method S10 of a cultured cartilage tissue material: Fig. 2A shows a state in which the bioabsorbable polymer porous body 11 is filled up with the cell suspension 12; and Fig. 2B shows a reversing step.
Fig. 3 is a view illustrating a part of a production method S20 of a cultured cartilage tissue material: Fig. 3A shows a temporary placement step; and Fig. 3B shows a state in which the bioabsorbable polymer porous body 11 is filled up with the cell suspension 12.
Fig. 4 is another view illustrating a part of the production method S20 of a cultured cartilage tissue material: Fig. 4A shows a sandwiching step; and Fig. 4B shows a holding step.
Fig. 5A is an enlarged planar view of the tissue material.
Fig. 5B is an enlarged cross-sectional view of the tissue material.
Fig. 6A is a view of tissue observed under a microscope.
Fig. 6B is a partially enlarged view of Fig. 6A.

### Modes for Carrying Out the Invention

The functions and benefits of the present invention described above will be apparent from the following modes for carrying out the invention. However, the invention is not limited to these embodiments.

A cultured cartilage tissue material according to one embodiment comprises: a bioabsorbable polymer porous body; and "chondrocytes" cultured in this bioabsorbable polymer porous body. Each of these will be described below. In the below description, a "stem cell that differentiates into chondrocyte" is sometimes referred to as a "chondrocyte differentiating stem cell".

The bioabsorbable polymer porous body is a porous body used at a time of seeding cells. At this time, the bioabsorbable polymer porous body has an interconnected porous structure with a pore diameter of 180 to 3500 µm and an average pore diameter of 350 to 2000 µm; and has a porosity of 60 to 95%. Additionally, the bioabsorbable polymer porous body is configured to have a compressive strength of 0.05 to 1 MPa. Herein, the pore diameter of the porous body does not take into account micropores of less than 10 µm that allow only liquid to pass therethrough, and means that in the whole porous body, 80% or more of the pores with a pore diameter of 10 µm or more have a pore diameter of 180 to 3500 µm. Note that the "porosity" is a value calculated from the weight of the bioabsorbable polymer porous body in comparison to the weight of a lump of raw material of the bioabsorbable polymer used, the bioabsorbable polymer porous body having the same volume as that of the lump of raw material of the bioabsorbable polymer used.

If the porosity is less than 60%, efficiency in culturing chondrocytes or chondrocyte differentiating stem cells, as described below, degrades. If it exceeds 95%, the strength of the bioabsorbable polymer porous body itself degrades. Therefore, the porosity is more preferably 80 to 90%. Additionally, if the pore diameter is less than 180 µm, it becomes difficult to introduce chondrocytes or chondrocyte differentiating stem cells into the bioabsorbable polymer porous body, therefore preventing sufficient seeding of the chondrocytes or chondrocyte differentiating stem cells into the bioabsorbable polymer porous body. On the other hand, if the pore diameter is more than 3500 µm, the strength of the bioabsorbable polymer porous body itself degrades.

Further, the average pore diameter is preferably 540 to 1200 µm.

As for the above compressive strength, if the compressive strength is less than 0.05 MPa, the bioabsorbable polymer porous body shrinks due to the extension stress of the chondrocytes or chondrocyte differentiating stem cells. On the other hand, it is technically difficult to make a bioabsorbable polymer porous body having a compressive strength of over 1.0 MPa. Note that the "compressive strength" herein refers to compressive fracture strength generated at a time of compressing a cylindrical test piece in a size of 10 mm diameter × 2 mm height at a cross head speed of 1 mm/min.

The whole shape of the bioabsorbable polymer porous body is not particularly limited, but it can be a shape that accords with a shape of a damaged site to be implanted. As a generally available bioabsorbable polymer porous body, various basic shapes such as cube, cuboid, hemisphere, and circular plate may be prepared. The thickness of the thinnest part of the porous body, (a dimension in a direction from the top to bottom of the porous body at the time of culturing) is preferably 2.2 to 100 mm, and more preferably 3 to 100 mm. If the thickness exceeds 100 mm, it becomes difficult to introduce cells into the porous body or to culture cells for a long period.

Any bioabsorbable polymer may be employed without particular limitations as long as it can maintain its configuration inside the living body for a certain period. An example thereof can be at least one selected from the followings, which have been conventionally used: polyglycolic acid; polylactic acid; a lactic acid-glycolic acid copolymer; poly-ε-caprolactone; a lactic acid-ε-caprolactone copolymer; polyamino acid; polyortho ester; and a copolymer thereof. Among these, polyglycolic acid, polylactic acid, and a lactic acid-glycolic acid copolymer are most preferred as being approved by US Food and Drug Administration (FDA) as a polymer which is harmless to the human body and in view of their actual performance. The weight average molecular weight of the bioabsorbable polymer material is preferably 5,000 to 2,000,000, and more preferably 10,000 to 500,000.

By using such a bioabsorbable polymer porous body, a cell suspension can properly permeate into the bioabsorbable polymer porous body 11, and the cells that need to be seeded can be introduced into the porous body, stably without being wasted, to be seeded therein. Then the cells are cultured, and thereby a cultured cartilage tissue material can be obtained.

The method of making a bioabsorbable polymer into a porous body is not particularly limited, but the following production method may be employed for example: mixing, in a substantially uniform manner, a particulate substance having a particle diameter of 100 to 2000 µm into a solution having a bioabsorbable polymer material dissolved in an organic solvent and freezing the mixture, the particulate substance not dissolving in this organic solvent but dissolving in a liquid that does not dissolve the bioabsorbable polymer material; thereafter drying the mixture to remove the organic solvent; thereby making a bioabsorbable polymer porous body which has a porous structure with a pore diameter of 5 to 50 µm and contains the particulate substance; pulverizing this bioabsorbable polymer porous body with a mill or the like; then removing the particulate substance by dissolving it in the liquid that does not dissolve the bioabsorbable polymer; thereafter sieving it to make the bioabsorbable polymer porous body into a granular form having an average particle size of 100 to 3000 µm; and then putting the granular bioabsorbable polymer porous material into a container having a predetermined shape to pressurize and heat it.

The production method S10 of a cultured cartilage tissue material (hereinafter sometimes referred to as a "production method S10") according to one embodiment will be described next.

The production method S10 comprises: an amplification culture step S11; a cell suspension preparation step S12; a tissue regeneration material production step S13; and a cartilage tissue culture step S14. Each of these steps will be described below.

Prior to the production method S10, cells are harvested. The cells to be harvested are chondrocytes or stem cells that differentiate into chondrocytes (hereinafter, the stem cell sometimes being referred to as a "chondrocyte differentiating stem cell"). As for the chondrocytes and the chondrocyte differentiating stem cells to be harvested, in the case of the chondrocytes, they may be used directly, and in the case of the chondrocyte differentiating stem cells, the following may be used: stem cells which can differentiate into chondrocytes or can promote restoration thereof, such as a marrow-derived mesenchymal stem cell, a mesenchymal cell, and a synovial cell. Examples thereof include cells harvested from: a bone marrow of a pelvis (an iliac bone) or of long bones of arms and legs (a thighbone, a tibia) ; and/or a bone marrow of periosteum, synovium, fat, alveolar bone etc.; a periosteum of a palate, an alveolar bone etc; or other sources.

As for the way to harvest these cells, methods ordinarily carried out in the medical setting may be employed without particular limitations. Particularly, such sources as the bone marrow of the iliac bone etc., and the periosteum of the palate, the alveolar bone, etc. are preferably employed as they enable an easy operation with a minimum exfoliation and incision of the skin and the muscle at the time of harvesting the cells.

Further, the chondrocytes and the chondrocyte differentiating stem cells to be harvested may be harvested not only from the person to be actually treated, but also from another person dead or alive, or an animal such as a cow, pig, horse, and chicken. However, when taking the cells from another person or an animal, in consideration of the possibility that the immunological rejection may occur, it is necessary to carry out, as a final step, an antigen removal treatment such as decellularization through rapid freezing by liquid nitrogen. This enables alleviation of distress at a person to be implanted.

The amplification culture step S11 is a step of culturing to amplify the chondrocytes or the chondrocyte differentiating stem cells that have been harvested as above, for one to two weeks using a culture dish for tissue culture by a known method. The culture medium to be used for culturing may be a known one; αMEM culture medium for cell culture which contains autologous serum or fetal bovine serum may be suitably employed. At this time, in a case of using mesenchymal stem cells, activating a specific growth factor (for example, bFGF) causes the mesenchymal stem cells to be proliferated with a high multiple differentiation potency thereof maintained, and thus can facilitate chondrogenic differentiation.

The cell suspension preparation step S12 is a step of preparing a cell suspension which contains the cells that have been cultured and amplified in the amplification culture step S11. In specific, the cell suspension is prepared by suspending the stem cells in a chondrogenic differentiation medium. The culture medium to be used herein, that is, the chondrogenic differentiation medium may be a known one. It is preferable for the cell suspension to have a cell concentration of 5 × 10⁶ to 1 × 10⁸ cells/ml.

The tissue regeneration material production step S13 is a step of producing a tissue regeneration material by introducing the cell suspension prepared in the cell suspension preparation step S12 into the bioabsorbable polymer porous body to seed and adhere the cells therein. The tissue regeneration material refers to a bioabsorbable polymer seeded with cells. The above described bioabsorbable polymer may be used herein.

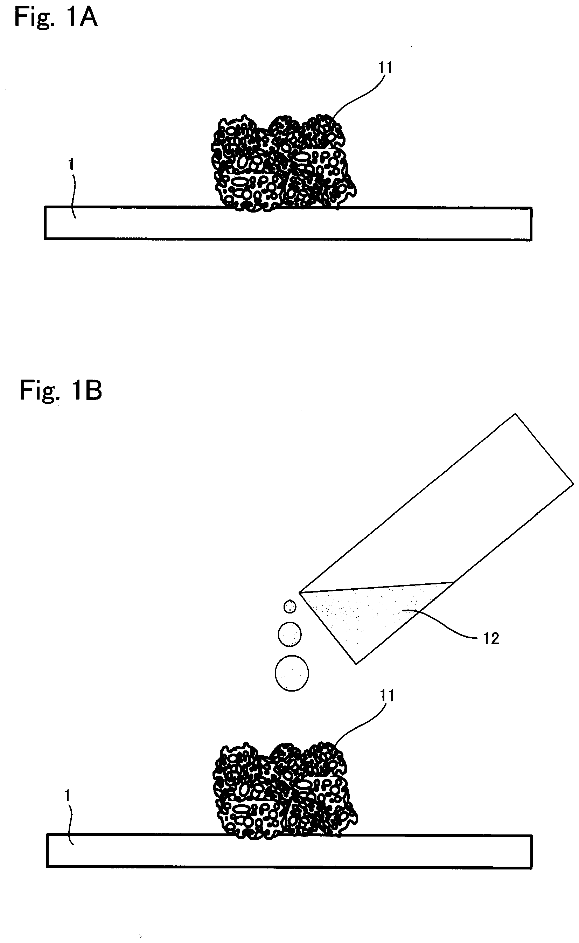

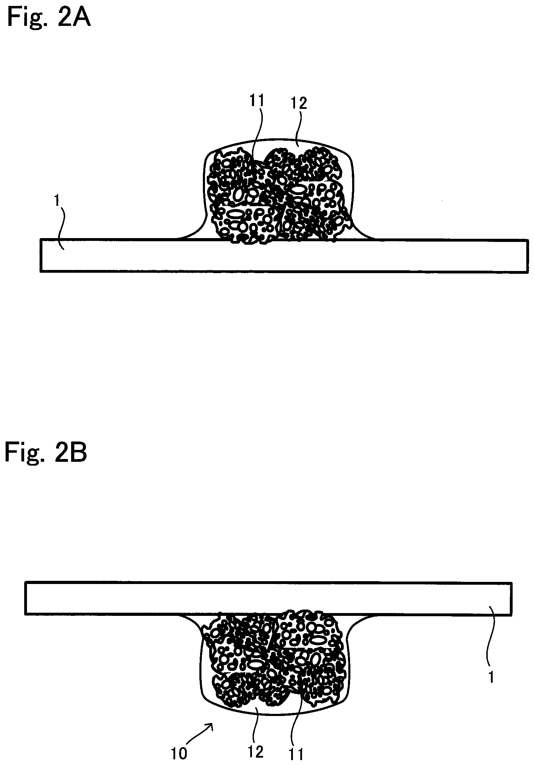

The tissue regeneration material production step S13 comprises: a placement step S131; a cell suspension introduction step S132; and a reversing step S133. Figs. 1 and 2 illustrate the tissue regeneration material production step S13.

The placement step S131 is a step of placing a bioabsorbable polymer porous body 11 on a holding plate 1, as shown in Fig. 1A. Herein, the holding plate 1 is preferably a plastic plate or a glass plate having a contact angle to water of 15 to 90°. Such a holding plate 1 can be obtained by processing, into a plate shape, a glass material used for a petri dish, flask, multiwell, etc., which have been conventionally used as a container for static culture of cells, or a plastic material such as polystyrene, polyethylene, and polyethylene terephthalate, etc. These materials sometimes have a high hydrophobic property. Therefore, it is preferable to modify the surface, as necessary, by a chemical treatment such as a plasma (corona discharge) treatment, to introduce a polar group and increase hydrophilicity of the surface to be contacted with the bioabsorbable polymer porous body 11, and to make the surface have a contact angle to water of 15 to 90°. Further, a ceramic coating may be formed on the surface to be contacted. It is preferable for the surface to be contacted to be smooth; however, it may have grooves or streaky protrusions or the like having a height of several hundred micrometers.

Through the placement step S131, the bioabsorbable polymer porous body 11 is arranged on the surface of the holding plate 1. Then in the cell suspension introduction step S132, the cell suspension 12 prepared in the cell suspension preparation step S12 is introduced into the bioabsorbable polymer porous body 11 for example by dripping or injection, as shown in Fig. 1B. Thereby, the cell suspension 12 permeates into the bioabsorbable polymer porous body 11 and fills up the entire porous body 11, as shown in Fig. 2A. At this time, if the amount of the cell suspension 12 is too large in relation to the volume of the bioabsorbable polymer porous body 11, it tends to be difficult to carry out the below described reversing step S133 or the culturing efficiency tends to degrade. Therefore, it is preferable to introduce the cell suspension in an amount that allows the entire bioabsorbable polymer porous body 11 to be immersed in the cell suspension and that does not cause excessive leakage of the cell suspension from the bioabsorbable polymer porous body 11.

Next, through the reversing step S133, the holding plate 1 and the bioabsorbable polymer porous body 11 containing the cell suspension 12 are reversed from the state shown in Fig. 2A, so that the holding plate 1 is on the upper side of the bioabsorbable polymer porous body 11 and the bioabsorbable polymer porous body 11 is below the holding plate 1, as shown in Fig. 2B. That is, when seen in the gravity direction, the holding plate 1 is on the upper side of the bioabsorbable polymer porous body 11, and they are kept still in the air in a state that the weight of the holding plate 1 is not applied to the bioabsorbable polymer porous body 11. With the holding plate 1 and the bioabsorbable polymer porous body 11 kept still in this state, the cells that have been introduced are adhered to the inner walls of the pores in the bioabsorbable polymer porous body 11, thereby completing seeding thereof. Thus obtained is the tissue regeneration material 10. The time to keep the holding plate 1 and the bioabsorbable polymer porous body 11 still in the air in order to adhere the cells to the bioabsorbable polymer porous body varies depending on the material of the bioabsorbable polymer porous body or the kinds of cells to be seeded; however, it is generally 20 to 300 minutes.

Further, when making the tissue regeneration material with the bioabsorbable polymer porous body 11 positioned below the holding plate 1 as in the present embodiment, the shape of the bioabsorbable polymer porous body 11 is not particularly limited as long as it is possible to: arrange the holding plate 1 on the upper side thereof through the reversing step S133 when seen in the gravity direction; keep them still in the air in a state that the weight of the holding plate 1 is not applied to the bioabsorbable polymer porous body 11; and thereby seed the cells in the bioabsorbable polymer porous body 11. However, at this time the area of the base (the area of the surface to contact with the holding plate 1) of the bioabsorbable polymer porous body 11 is preferably large enough in relation to the thickness thereof.

In specific, the area of the base is preferably 0.5 to 200 cm² in terms of the range of the volume 1 to 50000 cm³ of the bioabsorbable polymer porous body 11. If the area of the base is less than 0.5 cm² or if it exceeds 200 cm², it is difficult to seed the cells with the bioabsorbable polymer porous body hung on the holding plate.

Herein, if the bioabsorbable polymer porous body 11 has a relatively large pore, the cell suspension 12 can properly permeate into the bioabsorbable polymer porous body 11. Then by finally arranging the holding plate 1 on the bioabsorbable polymer porous body 11 when seen in the gravity direction, as above, and keeping them still in the air in the state that the weight of the holding plate 1 is not applied to the bioabsorbable polymer porous body 11, the cell suspension 12 can be retained in the bioabsorbable polymer porous body 11 so that it does not leak therefrom. Accordingly, it is possible to introduce the cell suspension 12 stably without wasting it and seed the cells into the bioabsorbable polymer porous body.

The cartilage tissue culture step S14 is a step of making a cultured cartilage tissue material from the tissue regeneration material 10 produced in the tissue regeneration material production step S13. In the cartilage tissue culture step S14, if the cells seeded in the tissue regeneration material 10 are chondrocytes, these are cultured to be amplified. If the cells seeded in the tissue regeneration material 10 are chondrocyte differentiating stem cells, these are differentiated into chondrocytes, which are then cultured to be thereby amplified.

Therefore, the cultured cartilage tissue material is formed from the tissue regeneration material 10 having gone through the culture process as above, so it comprises the bioabsorbable polymer and the cultured cartilage tissue being contained in this bioabsorbable polymer. That is, in the present embodiment, the cultured cartilage tissue material is a material made by culturing the chondrocytes or the chondrocytes having differentiated from the chondrocyte differentiating stem cells, which are contained in the above described tissue regeneration material.

A known method may be used to amplify/differentiate (culture) the chondrocytes. For example, the chondrocytes can be amplified by a proliferation culture medium. A chondrogenic differentiation medium may also be used to differentiate and culture the chondrocytes after they are proliferated.

Next, the production method S20 of a cultured cartilage tissue material (hereinafter sometimes being referred to as a "production method S20") according to another embodiment will be described. In the present embodiment, the same elements as those described in the above production method S10 are given the same numerals, and descriptions thereof are omitted.

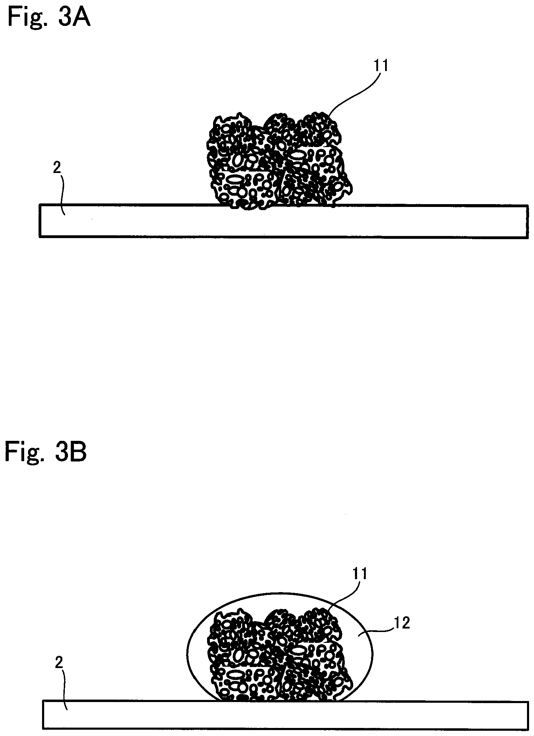

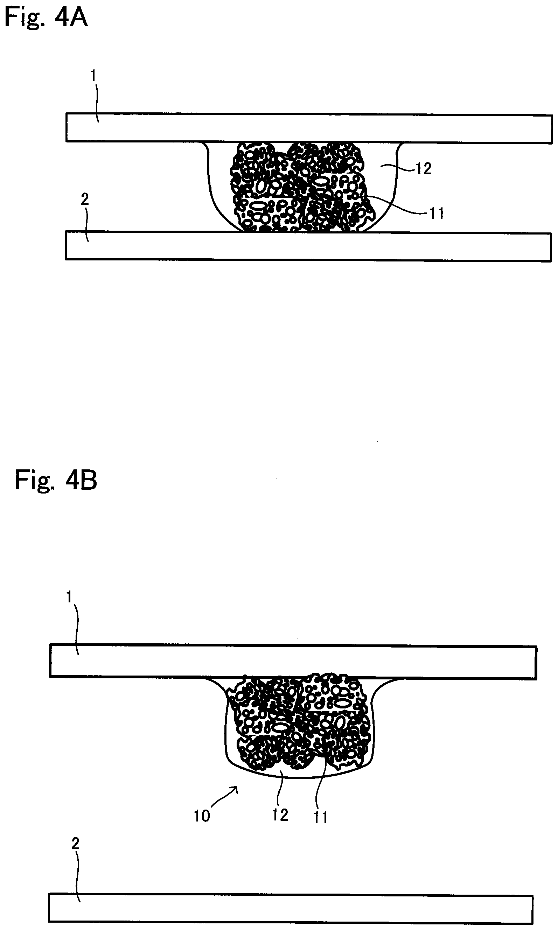

The production method S20 comprises a tissue regeneration material production step S23, instead of the tissue regeneration material production step S13 of the production method S10. The tissue regeneration material production step S23 comprises: a temporary placement step S231; a cell suspension introduction step S232; a sandwiching step S233; and a holding step S234. Figs. 3 and 4 illustrate the tissue regeneration material production step S23.

The temporary placement step S231 is a step of placing the bioabsorbable polymer porous body 11 onto a temporary placement plate 2, which is a plate member having high water repellency, as shown in Fig. 3A. The temporary placement plate 2 is preferably a plastic plate or a glass plate having a contact angle to water of more than 90°.

In the cell suspension introduction step S232, the cell suspension 12 is introduced into the bioabsorbable polymer porous body 11, as in the above described cell suspension introduction step S132. Through this, the cell suspension 12 permeates into the bioabsorbable polymer porous body 11 to fill up the whole bioabsorbable polymer porous body 11, as shown in Fig. 3B.

The sandwiching step S233 is a step of contacting the bioabsorbable polymer porous body 11 filled up with the cell suspension to a surface of the holding plate 1 in a manner sandwiching the bioabsorbable polymer porous body 11 between the holding plate 1 and the temporary placement plate 2, as shown in Fig. 4A.

The holding step S234 is a step of pulling up the holding plate 1 contacted with the bioabsorbable polymer porous body 11, holding the bioabsorbable polymer porous body 11 on the holding plate 1 side, and releasing it from the temporary placement plate 2, as shown in Fig. 4B. Through this, as in one embodiment described above, the holding plate 1 is arranged to be on the upper side of the bioabsorbable polymer porous body 11 when seen in the gravity direction, and they are kept still in the air in a state that the weight of the holding plate 1 is not applied to the bioabsorbable polymer porous body 11. With the holding plate 1 and the bioabsorbable polymer porous body 11 kept still in this state, the cells that have been introduced into the bioabsorbable polymer porous body 11 are adhered to the inner wall thereof, thereby completing seeding of the cells. Thus obtained is the tissue regeneration material 10.

Herein, the temporary placement plate 2 is constituted by a water-repellent plate; and the holding plate 1 is constituted by a hydrophilic plate. Therefore, the above described holding and releasing of the bioabsorbable polymer porous body filled with the cell suspension can be properly done.

The cultured cartilage tissue material according to the present invention is a mixed body of chondrocytes in a concentration 1 × 10⁷ to 1 × 10⁹ cells/cm³, and a bioabsorbable polymer. A thickness of the thinnest part of the cultured cartilage tissue material is 2.2 to 100 mm. A volume ratio between the chondrocytes and the bioabsorbable polymer is 7:3 to 9.5:0.5. A production amount of glycosaminoglycan is 0.001 to 0.2 ng per unit cell. A content ratio between type I collagen and type II collagen is 10:90 to 1:99. A content of type II collagen is 0.01 to 0.65 mg per 1 mg in dry weight of the tissue. Such a concentration of the chondrocytes, which is 1 × 10⁷ to 1 × 10⁹ cells/cm³, is substantially the same as in the cartilage of the living body, and thus is larger than the concentration in the conventional materials for cartilage tissue regeneration.

In the cultured cartilage tissue material according to the present invention, the volume ratio of the cartilage tissue and the bioabsorbable polymer is 7:3 to 9.5:0.5. If the volume ratio is less than 7:3 in terms of the cartilage tissue, an influence of the bioabsorbable polymer becomes large; and therefore, due to an influence by the decomposition product or small number of cells, the efficiency of engraftment in the living body degrades. On the other hand, if the volume ratio exceeds 9.5:0.5 in terms of the cartilage tissue, the bioabsorbable polymer cannot maintain its intended shape, causing formation of a shrunk mass of cells and necrosis of the cells inside.

In the cultured cartilage tissue material according to the present invention, the production amount of glycosaminoglycan is 0.001 to 0.2 ng per unit cell; the content ratio between type I collagen and type II collagen is 10:90 to 1:99; and the content of type II collagen is 0.01 to 0.65 mg per 1 mg in dry weight of the tissue. These are approximately the same as in the cartilage in the living body.

According to the cultured cartilage tissue material described above, a cartilage tissue material which is large in volume can be made, and implanting this into a patient enables repair of a cartilage defect which is large in volume. Therefore, even when the cartilage has a large damage, it can be regenerated within a short time by being implanted with this cartilage tissue material. In specific, bulk-form cartilage in large size equivalent to the size of the bioabsorbable polymer porous body is made and implanted into a damaged site, thereby regenerating cartilage large in volume. Further, the cartilage in bulk form may be pulverized into powder form to be easily applied to the implantation site, and then this powder-form cartilage may be implanted in the site.

The cultured cartilage tissue material according to the present invention may not only be used as cartilage but also may be implanted in a damaged site of bone caused by a bone-related disease to regenerate the bone through enchondral ossification in the living body. In this case as well, the bioabsorbable polymer disappears with time.

### Examples

Examples of the present invention will be described below.

### <Example 1>

In Example 1, a cultured cartilage tissue material D1 was made through the following process, and implanted in the implantation step.

### (Preparation of Cells A1 through the Cell Harvesting Step and the Amplification Culture Step)

Cells obtained by giving a collagenase treatment to the tissue harvested from a human synovial membrane were suspended in an αMEM culture medium supplemented with 20% FBS at a concentration of 1 × 10⁴ cells/ml nucleated cells. Thereafter, 10 ml thereof was seeded on a 10 cm diameter culture dish. The cells were cultured to be proliferated under the presence of 5% carbon dioxide gas at 37°C. The culture medium was changed on the third day, to remove non-adherent cells (hematopoietic cells) . After that, the culture medium was changed every three days. From the fifth day, bFGF was added to the culture medium in an amount of 3 ng/ml. Around the 10th day, the cells were proliferated to be nearly confluent. These culture dishes were incubated for 5 minutes with trypsin (0.05%) and EDTA (0.2 mM), to isolate the cells. The number of cells was measured by a Coulter counter (ZI single, manufactured by Coulter Corporation), and the cells were seeded at a density of 5000 cells/cm². This operation was repeated, and the third passage cells obtained from the second subculture dish nearly confluent were used.

Thereby, the cells A1 being the mesenchymal stem cells taken from the human synovial membrane, were obtained.

### (Preparation of Bioabsorbable Polymer Porous Body B1)

A DL-lactic acid/glycolic acid copolymer having a molecular weight of 250,000 was dissolved in dioxane, and thereafter the solution was mixed with sodium chloride having a particle size of around 500 µm. Then the mixture was freeze-dried, and was pulverized and desalinated to thereby obtain a powder-form material. The powder-form material thus obtained was molded by compression heating and was γ-sterilized. Thereby, a bioabsorbable polymer porous body B1 being a block of a polylactic acid/glycolic acid copolymer (PLGA) was obtained, the bioabsorbable polymer porous body being in a disc block shape, having a porous structure, being made of a bioabsorbable synthetic polymer, and having an average pore diameter of 540 µm, a porosity of 90%, a compressive strength of 0.2 MPa, a diameter of 9 mm and a thickness of 3 mm.

### (Production of a Tissue Regeneration Material C1 through the Cell Suspension Preparation Step and the Tissue Regeneration Material Production Step)

The above bioabsorbable polymer porous body B1 was placed onto a plasma-treated polystyrene 60 mm culture dish (a contact angle to water of 70°), with the disc-shaped bottom surface of the bioabsorbable polymer porous body B1 contacted to the upper surface of the plasma-treated polystyrene culture dish. The cells A1, which were suspended in a chondrogenic differentiation medium (αMEM; glucose 4.5 mg/ml; 10⁻⁷ M dexamethasone; 50 µg/ml ascorbic acid-2-phosphoric acid; 10 ng/ml TGF-β; 6.25 µg/ml insulin; 6.25 µg/ml transferrin; 6.25 ng/ml selenic acid; 5.33 µg/ml linoleic acid; 1.25 mg/ml bovine serum albumin) at a density of 2 × 10⁷ cells/ml, were introduced into the bioabsorbable polymer porous body B1 in an amount of 0.19 ml by dripping. With the culture dish reversed to be upside down, the cells A1 were seeded on the bioabsorbable polymer porous body B1 by adhering the cells to the material for 100 minutes under the conditions of 37°C, 100% humidity, and 5% CO₂, to thereby obtain a tissue regeneration material C1.

### (Production of a Cultured Cartilage Tissue Material D1 through the cartilage tissue culture step)

Thereafter, the tissue regeneration material C1 was put into a 50 ml centrifuge tube filled with a chondrogenic differentiation cell fluid, and the cells were cultured at 37°C for four weeks while changing the culture medium every three days. Thereby, a cultured cartilage tissue regeneration material D1 was produced.

The tissue material thus obtained had a diameter of 8.8 mm and a thickness of 2.8 mm. Further, the tissue material was evaluated histopathologically and found to be a mature cartilage tissue with a cartilage matrix deposited in a large amount. In addition, a papain-digested sample was quantitated by a GAG quantification kit and a DNA quantification kit to determine the amount of glycosaminoglycan per unit DNA; and the amount of glycosaminoglycan per unit cell was quantitated from a separately obtained calculation result of the relation between the cell number and the DNA amount. Type II collagen and type I collagen were quantitated by ELISA, and determined in terms of unit cell in the same manner. Furthermore, the cultured cartilage tissue was observed under a microscope, and the size "length × width × height" thereof was measured to calculate the volume thereof. According to the above results, the amount of glycosaminoglycan per unit cell was 0.03 ng, and the ratio between type I collagen and type II collagen was 1:99. The cell density in the tissue was 8 × 10⁷ cells/cm³.

### (Implantation by the Implantation Step and the Evaluation Thereof)

The cultured cartilage tissue material D1 was implanted under the skin of the back of a SCID mouse. Then the implant was collected after four weeks had passed, to be evaluated histopathologically. The result confirmed that the implant was a mature cartilage tissue with a cartilage matrix deposited in a large amount and that Example 1 enabled regeneration of ectopic bone.

### <Example 2>

In Example 2, a cultured cartilage tissue material D2 was made through the following process, and implanted in the implantation step.

### (Preparation of Cells A2 through the Cell Harvesting Step and the Amplification Culture Step)

A rabbit iliac bone marrow was suspended in an αMEM culture medium supplemented with 10% FBS at a concentration of 1 × 10⁴ cells/ml nucleated cells. Thereafter, 10 ml thereof was seeded on a 10 cm diameter culture dish. The cells were cultured to be proliferated under the presence of 5% carbon dioxide gas at 37°C. The culture medium was changed on the third day, to remove non-adherent cells (hematopoietic cells). After that, the culture medium was changed every three days. From the fifth day, bFGF was added to the culture medium in an amount of 3 ng/ml. Around the 10th day, the cells were proliferated to be nearly confluent. These culture dishes were incubated for 5 minutes with trypsin (0.05%) and EDTA (0.2 mM), to isolate the cells. The number of cells was measured by a hemocytometer, and the cells were seeded at a density of 5000 cells/cm². This operation was repeated, and the third passage cells obtained from the second subculture dish nearly confluent were used.

Thereby, the cells A2 being the mesenchymal stem cells harvested from the rabbit iliac bone marrow, were obtained.

### (Preparation of Bioabsorbable Polymer Porous Body B2)

A DL-lactic acid/glycolic acid copolymer having a molecular weight of 250,000 was dissolved in dioxane, and thereafter the solution was mixed with sodium chloride having a particle size of around 500 µm. Then the mixture was freeze-dried, and was pulverized and desalinated to thereby obtain a powder-form material. The powder-form material thus obtained was molded by compression heating and was γ-sterilized. Thereby, a block-shaped bioabsorbable polymer porous body B2 being a block of a polylactic acid/glycolic acid copolymer (PLGA) was obtained, the bioabsorbable polymer porous body being made of a bioabsorbable synthetic polymer material, and having an average pore diameter of 600 µm, a porosity of 80%, a compressive strength of 0.6 MPa, and 5.3 mm diameter × 2.5 mm height.

### (Production of a Tissue Regeneration Material C2 through the Cell Suspension Preparation Step and the Tissue Regeneration Material Production Step)

The bioabsorbable polymer porous body B2 was placed onto a plasma-treated polystyrene 60 mm culture dish (a contact angle to water of 70°), with the bottom surface of the bioabsorbable polymer porous body B2 contacted to the upper surface of the plasma-treated polystyrene culture dish. The cells A2, which were suspended in a chondrogenic differentiation medium (αMEM; glucose 4.5 mg/ml; 10⁻⁷ M dexamethasone; 50 µg/ml ascorbic acid-2-phosphoric acid; 10 ng/ml TGF-β; 6.25 µg/ml insulin; 6.25 µg/ml transferrin; 6.25 ng/ml selenic acid; 5.33 µg/ml linoleic acid; 1.25 mg/ml bovine serum albumin) at a density of 2 × 10⁷ cells/ml, were introduced into the bioabsorbable polymer porous body B2 in an amount of 0.15 ml by dripping. With the culture dish reversed to be upside down, the cells A2 were seeded on the bioabsorbable polymer porous body B2 by adhering the cells to the material for 100 minutes under the conditions of 37°C, 100% humidity, and 5% CO₂, to thereby obtain a tissue regeneration material C2.

### (Production of a Cultured Cartilage Tissue Material D2 through the cartilage tissue culture step)

Thereafter, the tissue regeneration material C2 was put into a 50 ml centrifuge tube filled with a chondrogenic differentiation cell fluid, and the cells were cultured at 37°C for four weeks while changing the culture medium every three days. Thereby, a cultured cartilage tissue regeneration material D2 was produced.

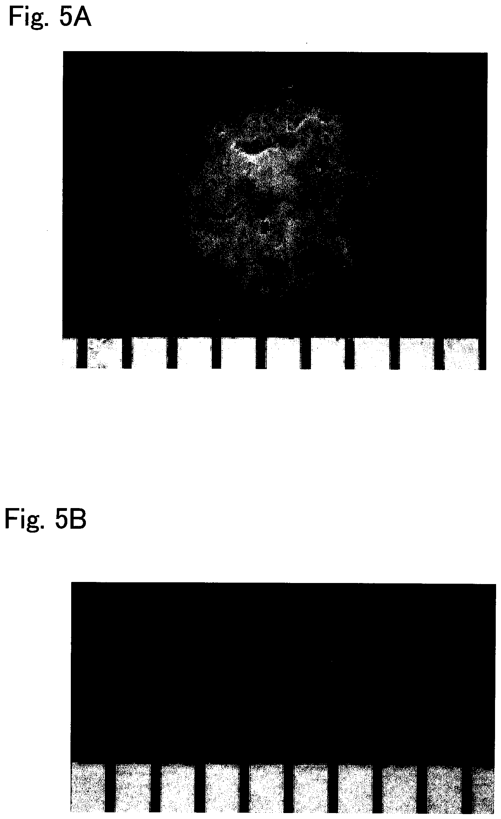

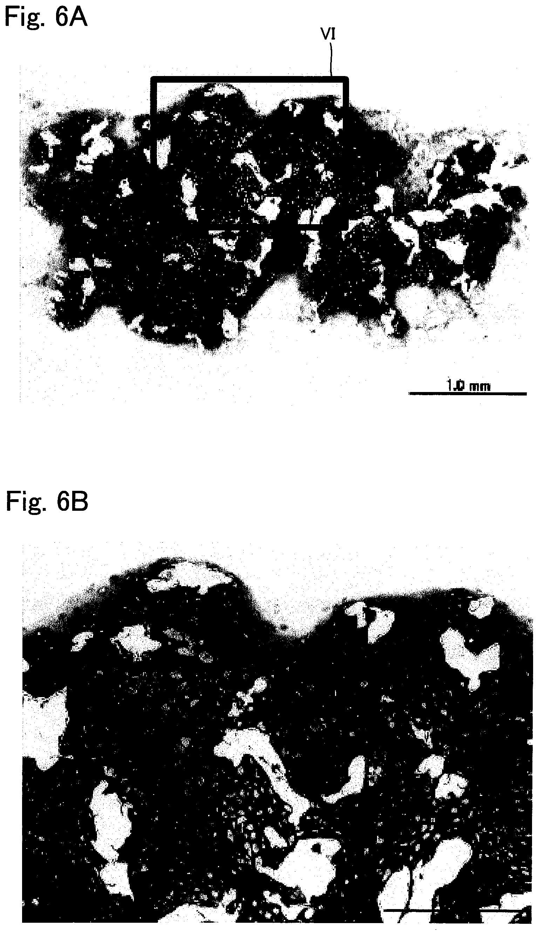

The tissue material thus obtained had a diameter of 5 mm and a thickness of 2.3 mm. Fig. 5 shows an enlarged view of the tissue material obtained. Fig. 5A is a planar view, and Fig. 5B is a cross-sectional view. Additionally, the tissue was fixed in a 10% formalin neutral buffer solution and embedded in paraffin; and was cut into a thickness of 5 µm by a microtome. A cross section of the tissue was toluidine blue stained and observed under a light microscope; and was found to be a mature cartilage tissue having a large amount of metachromasia-positive cartilage matrix which is stained in red-purple color by toluidine blue stain. Fig. 6A shows the tissue observed under a microscope. Fig. 6B is an enlarged view of the part of Fig. 6A taken by VI. In addition, a papain-digested sample was quantitated by a GAG quantification kit and a DNA quantification kit to determine the amount of glycosaminoglycan per unit DNA; and the amount of glycosaminoglycan per unit cell was quantitated from a separately obtained calculation result of the relation between the cell number and the DNA amount. Type II collagen and type I collagen were quantitated by ELISA, and likewise determined in terms of unit cell. Furthermore, the cultured cartilage tissue was observed under a microscope, and the size "length × width × height" thereof was measured to calculate the volume thereof. According to the above results, the amount of glycosaminoglycan per unit cell was 0.03 ng, and the ratio between type I collagen and type II collagen was 1:99. The cell density in the tissue was 8 × 10⁷ cells/cm³.

### (Implantation by the Implantation Step and the Evaluation Thereof)

A defect of cartilage in 6 mm diameter × 3 mm was created in a knee joint part of a rabbit femur, to obtain a sample; and the cultured cartilage tissue material D2 was implanted into the model. A healing process of the defect part was evaluated histopathologically four weeks after the implantation. The result confirmed that a continuous cartilage tissue was regenerated in the knee joint part and that the treatment method according to Example 2 was a treatment method which enabled regeneration of cartilage in the knee joint part of the femur.

### Description of the Reference Numerals

- 1: holding plate
- 2: temporary placement plate
- 10: tissue regeneration material
- 11: bioabsorbable polymer porous body
- 12: cell suspension

## Claims

1. A cultured cartilage tissue material to be used for cartilage regeneration comprising chondrocytes,
wherein the cultured cartilage tissue material is a mixed body of the chondrocytes in a concentration of 1 × 10⁷ to 1 × 10⁹ cells/cm³, and a bioabsorbable polymer;
a thickness of the thinnest part of the cultured cartilage tissue material is 2.2 to 100 mm;
a volume ratio between the chondrocytes and the bioabsorbable polymer is 7:3 to 9.5:0.5;
a production amount of glycosaminoglycan is 0.001 to 0.2 ng per unit cell;
a content ratio between type I collagen and type II collagen is 10:90 to 1:99; and
a content of type II collagen is 0.01 to 0.65 mg per 1 mg of dry weight of tissue.

2. A cultured cartilage tissue material as claimed in claim 1, wherein an area of the base of the cultured cartilage tissue material is 0.5 cm² to 200 cm².

## Patentansprüche

1. Gezüchtetes Knorpelgewebematerial zur Verwendung zur Knorpelregeneration, Chondrozyten umfassend,
wobei das gezüchtete Knorpelgewebematerial ein gemischter Körper aus Chondrozyten in einer Konzentration von 1 x 10⁷ bis 1 x 10⁹ Zellen/cm³ und eines bioabsorbierbaren Polymers ist;
eine Dicke des dünnsten Teils des gezüchteten Knorpelgewebematerials 2,2 bis 100 mm beträgt;
ein Volumenverhältnis zwischen den Chondrozyten und dem bioabsorbierbaren Polymer zwischen 7:3 und 9,5:0,5 liegt;
eine Produktionsmenge von Glycosaminoglycan zwischen 0,001 und 0,2 ng je Zelleneinheit beträgt;
ein Inhaltsverhältnis zwischen Kollagen Typ I und Kollagen Typ II zwischen 10:90 und 1:99 liegt; und
ein Anteil von Kollagen Typ II 0,01 bis 0,65 mg pro 1 mg Gewebetrockengewicht beträgt.

2. Gezüchtetes Knorpelgewebematerial nach Anspruch 1, wobei eine Fläche der Basis des gezüchteten Knorpelgewebematerials zwischen 0,5 cm² und 200 cm² liegt.

## Revendications

1. Matériau de tissu cartilagineux en culture à utiliser pour la régénération du cartilage comprenant des chondrocytes,
le matériau de tissu cartilagineux en culture étant un corps mixte des chondrocytes à une concentration de 1 x 10⁷ à 1 x 10⁹ cellules/cm³, et un polymère bioabsorbable ;
une épaisseur de la partie la plus mince du tissu cartilagineux en culture étant comprise entre 2,2 et 100 mm ;
un rapport volumique entre les chondrocytes et le polymère bioabsorbable étant compris entre 7:3 et 9,5:0,5 ;
une quantité de production de glycosaminoglycane étant comprise entre 0,001 et 0,2 ng par cellule unitaire ;
un rapport de teneur entre le collagène de type I et le collagène de type II étant compris entre 10:90 et 1:99 ; et
une teneur en collagène de type II étant comprise entre 0,01 et 0,65 mg pour 1 mg de poids sec de tissu.

2. Matériau de tissu cartilagineux en culture selon la revendication 1, une surface de la base du matériau de tissu cartilagineux en culture étant comprise entre 0,5 cm² et 200 cm².
